# EUROPÄISCHE PATENTSCHRIFT

(11) **EP 2 117 425 B1**
(45) Veröffentlichungstag und Bekanntmachung des Hinweises auf die Patenterteilung: **02.04.2014**
(21) Anmeldenummer: 08700873.6
(22) Anmeldetag: 19.01.2008
(51) Int. Cl.: A61B 5/0476

(54) **Phantom mit Dipole**
Phantom with dipoles
Fantôme avec dipoles

(30) Priorität: 16.02.2007 DE 102007007686
(43) Veröffentlichungstag der Anmeldung: 18.11.2009
(73) Patentinhaber: Forschungszentrum Jülich GmbH, 52425 Jülich (DE)
(72) Erfinder: BOERS, Frank, 41812 Erkelenz (DE); DAMMERS, Jürgen, 52428 Jülich (DE); RONGEN, Heinz, 52353 Düren (DE); SCHIEK, Michael, 52066 Aachen (DE)
(86) Internationale Anmeldenummer: PCT/DE2008/000089
(87) Internationale Veröffentlichungsnummer: WO 2008/098538

(56) Entgegenhaltungen:
- JP-A- 4 061 844
- JP-A- 2001 070 272
- US-A- 5 099 144
- LEAHY R M ET AL: "A study of dipole localization accuracy for MEG and EEG using a human skull phantom" ELECTROENCEPHALOGRAPHY AND CLINICAL NEUROPHYSIOLOGY ELSEVIER IRELAND, Bd. 107, Nr. 2, August 1998 (1998-08), Seiten 159-173, XP002488488 ISSN: 0013-4694
- FRISTON K J: "Brain function, nonlinear coupling, and neuronal transients." THE NEUROSCIENTIST : A REVIEW JOURNAL BRINGING NEUROBIOLOGY, NEUROLOGY AND PSYCHIATRY OCT 2001, Bd. 7, Nr. 5, Oktober 2001 (2001-10), Seiten 406-418, XP002488489 ISSN: 1073-8584 in der Anmeldung erwähnt

## Beschreibung

Die Erfindung betrifft ein Phantom.

Die Elektroenzephalographie, abgekürzt EEG, ist eine wichtige Untersuchungsmethode zur Charakterisierung der Gehirntätigkeit. Dabei werden schwache elektrische Ströme, welche die Gehirntätigkeit-begleiten, an definierten Punkten der Kopfhaut mittels Elektroden abgeleitet. Die Spannungsschwankungen zwischen jeweils zwei dieser Elektroden werden verstärkt und von einem Mehrkanalschreiber als Funktion der Zeit aufgezeichnet. Das so entstehende Elektroenzephalogramm lässt Rückschlüsse auf Gehirnerkrankungen zu.

Bei der Magnetoenzephalographie, abgekürzt MEG, wird hingegen eine Messung der magnetischen Aktivität des Gehirns durch äußere Sensoren, wie z. B. mittels sogenannter Superconducting Quantum Interference Devices (SQUIDs), vorgenommen. Die magnetischen Signale des Gehirns werden durch die elektrischen Ströme aktiver Nervenzellen verursacht. Daher kann man mit einem Magnetoenzephalograph Daten aufzeichnen, die ohne zeitliche Verzögerung Ausdruck der momentanen Gesamtaktivität des Gehirns sind.

Ein Magnetoenzephalograph zeigt gute räumliche und sehr hohe zeitliche Auflösung. Moderne Ganzkopf-Magnetoenzephalographen verfügen über eine helmartige Anordnung, umfassend z. B. etwa 300 Magnetfeldsensoren, welche während der Messung auf dem Kopf des Patienten bzw. Probanden berührungsfrei aufgesetzt wird. Da die magnetischen Signale des Gehirns nur wenige Femtotesla betragen, müssen äußere Störungen möglichst gut abgeschirmt werden.

Die leichte Anwendbarkeit des Gerätes bei gleichzeitig hoher Kanalzahl und genau bekannten Sensorpositionen sowie die durch die Messmodalität bedingte Möglichkeit, auch die Aktivität tiefer liegender Hirnregionen zu registrieren, sind die wichtigsten Vorteile der Magnetoenzephalographie im Vergleich zur Elektroenzephalographie.

Aufgezeichnete Gehirnsignale stellen in der Regel eine komplexe Komposition vieler sich überlagernder einzelner Gehirnaktivitäten und auch körpereigener Artefaktsignale, wie z. B. der Herzaktivität und der Augen und Gesichtsmuskeln, dar. Die Isolation und Lokalisation der den wesentlichen Gehirnaktivitäten zugehörigen Signalen aus allen ermittelten Sensorsignalen, um diese gezielt untersuchen zu können, stellt eine besondere Herausforderung in der modernen Neurowissenschaft dar.

Eine Schwierigkeit bezüglich der Lokalisation von elektrischen Strömen mittels der durch ein Magnetoenzephalographen gemessenen Magnetfelder besteht in der Nichteindeutigkeit des so genannten inversen Problems. Danach kann ein und dieselbe beliebig genau gemessene Magnetfeldverteilung durch verschiedene Anordnungen elektrischer Stromdichten erzeugt werden. Für die Lokalisation der Stromdichten anhand der gemessenen Magnetfelder sind demnach noch einschränkende Annahmen über die geometrische Verteilung der Stromdichten notwendig. Die unterschiedlichen Rückrechnungsalgorithmen basieren auf Annahmen.

Aus Phillips (Phillips, J.W., Leahy, R.M., Mosher, J.C. (1997). MEG-based imaging of focal neuronal current sources. IEEE Transactions on medical imaging, Vol. 16, No. 3, 338-348) sind verschiedene Rückrechnungsalgorithmen bekannt.

Die Güte von Rückrechnungsalgorithmen bezüglich der Lokalisation und der Quantifizierung der Stromdichten wird mittels künstlich erzeugter Stromdichteverteilungen vorgenommen. In einem so genannten Phantom werden hierzu elektrische oder magnetische Dipole definierter Stärke an lokal exakt definierten physikalischen Orten erzeugt. Ein Phantom stellt somit eine Vorrichtung zur Erzeugung räumlich verteilter elektromagnetischer Signale dar.

Ein Kopfphantom umfasst hierzu beispielsweise ein Array aus 32 Stromdipolen, einen PC zur Steuerung eines 32-Kanal Dipol-Treibers sowie das eigentliche Kopfphantom selbst. Ein derartiges Phantom ist der Druckschrift Spencer et al. (Spencer, M.E., Leahy, R.M, and Mosher, J.C., 1996. A skull-based multiple dipole phantom for EEG and MEG Studies. Proceedings of the 10th international conference on biomagnetism, Biomag '96, Santa Fe, New Mexico, Februar 1996) entnehmbar.

Ein elektrischer Dipol wird in Phantomen mittels eines dünnen Koaxialkabels erzeugt, welches über eine Spannungsquelle angesteuert wird. Die beiden Kontakte des Kabels enden offen in einem elektrisch leitenden Medium des Phantoms. Sind die Enden durch einen als Spule gewickelten Drahtes miteinander verbunden, wird auf diese Weise ein magnetischer Dipol erzeugbar. Auf diese Weise bildet jedes Koaxialkabel einen Messort aus, an dem nach Anlegen einer Spannung ein Dipol an diesem exakt definierten Punkt erzeugt wird. Der Dipol wird durch äußere Sensoren erfasst.

Eine Vielzahl an Dipolen ist im Phantom über eine entsprechende Anzahl an räumlich verteilten Koaxialkabeln, welche elektrisch über einen Dipoltreiber angesteuert werden, erzeugbar.

Ein Kopfphantom ist zur Nachbildung der Anatomie eines Kopfes kopfförmig ausgebildet. Ein elektrisch leitendes Medium im Innern des Phantoms ist der elektrischen Eigenschaft des Gehirns in etwa nachgebildet, um eine Kalibrierung der im Probanden gemessenen Daten an Hand der im Phantom ermittelten Daten zu ermöglichen.

Regelmäßig treten Abweichungen der mittels der Algorithmen rekonstruierten Verteilungen von den tatsächlichen Verteilungen bereits für ein Phantom auf. Je geringer die Abweichung der rekonstruierten von den tatsächlichen Parametern, wie Stärke und Lokalisation der Verteilungen, ist, umso höher ist die Güte des Gesamtmesssystems. Die Abweichungen der Daten für einzelne Kanäle können in eine iterative Optimierung des Rückrechnungsalgorithmus einfließen.

Um die Güte des Messsystems nachhaltig zu verbessern, muss die Messung der Stromdichteverteilungen bzw. der Magnetfeldverteilungen möglichst störungsfrei erfolgen. Zu diesem Zweck weisen die verwendeten Koaxialkabel je nach Anforderungen qualitativ mehr oder weniger hochwertige Abschirmungen auf. Zudem ist es möglich, nur einen Kanal gleichzeitig im Phantom anzusprechen.

Nachteilig bei dieser Vorgehensweise ist aber, dass dann nur ein einziger Dipol im Phantom gleichzeitig erzeugt wird. Die Kalibrierung des Messsystems wird dadurch bedingt vergleichsweise zeitaufwendig und komplexe Fragestellungen können nicht realitäts- und zeitnah simuliert werden.

Aus Friston (Karl J. Friston, 2001. The Neuroscientist, Vol. 7, No. 5, 406-418. SAGE Publications Brain Function, Nonlinear Coupling, and Neuronal Transients), ist bekannt, dass neben der Lokalisation und quantitativen Beschreibung der neuronalen elektrischen Aktivität die Analyse der Interaktionen verschiedener Hirnareale miteinander von zentraler Bedeutung sowohl für die moderne Hirnforschung als auch für die klinische Neurologie geworden ist. Ein Kernpunkt moderner neurologischer Fragestellung ist daher neben der Art der Kopplung der Areale (linear oder nichtlinear), insbesondere auch die Direktionalität der Kopplung. Die Direktionalität der Kopplung beschreibt, welches Hirnareal von der Aktivität eines anderen, mit diesem in Verbindung stehenden Areal, beeinflusst wird.

Nachteilig ist die Güte der im Stand der Technik beschriebenen Gesamtmesssysteme trotz Fortschritten in der Auslegung der Phantome sowie der Modellierung der Messdaten nach wie vor als nicht ausreichend für derartig komplexe Fragestellungen, und insbesondere nicht für die Beschreibung der nichtlinearen Transferfunktion eines Messsystems, zu bezeichnen.

Die US 5099144 A offenbart ein Phantom mit einem ersten Mittel zur Erzeugung mindestens eines Dipols, wobei mindestens ein zweites Mittel zur Ansteuerung des mindestens ersten Mittels auf nicht elektrische Weise vorhanden ist.

Aufgabe der Erfindung ist es daher, ein Phantom bereit zu stellen, welches die aus dem Stand der Technik bekannten Nachteile nicht aufweist, so dass auch komplexe Fragestellungen, wie die Beschreibung der nichtlinearen Transferfunktion des Messsystems, untersucht werden können.

Die Aufgabe wird durch ein Verfahren gemäß Hauptanspruch gelöst. Vorteilhafte Ausgestaltungen ergeben sich aus den darauf rückbezogenen Patentansprüchen.

Das Phantom umfasst mindestens ein erstes Mittel zur Erzeugung mindestens eines Dipols. Es ist gekennzeichnet durch mindestens ein zweites Mittel zur Ansteuerung des mindestens einen ersten Mittels auf nicht elektrische Weise. Vorteilhaft wird dadurch bewirkt, dass der Dipol störungsfrei vom ersten Mittel erzeugt wird, da im Gegensatz zu Phantomen gemäß Stand der Technik auf elektrische Zuleitungen verzichtet wird.

Im Rahmen der Erfindung wurde erkannt, dass Störungen in Form unerwünschter elektromagnetischer Felder auch durch hohen Aufwand bei der Abschirmung der Koaxialkabel nicht vermeidbar sind. Störungen durch elektrische Zuleitungen verzerren auch bei den qualitativ hochwertigsten Abschirmungen die durch die Dipole erzeugten elektromagnetischen Felder und erschweren deren genaue Lokalisation sowie die Abschätzung deren Stärke.

Es wurde weiterhin im Rahmen der Erfindung erkannt, dass diese Störungen mit der Anzahl und der Stärke der gleichzeitig erzeugten Dipole an den Zuleitungen ansteigt, so dass die Genauigkeit der benötigten Güteabschätzung hinsichtlich der Lokalisation, der quantitativen Beschreibung und der Rekonstruktion der komplexen zeitlichen Dynamik der Stromdichteverteilungen beeinträchtigt ist. Dadurch ist bei gleichzeitiger Ansteuerung der Dipole über die elektrischen Zuleitungen im Phantom die Nachweisempfindlichkeit des Messsystems insbesondere hinsichtlich schwacher nichtlinearer Kopplungen mit oder ohne eine zeitlich wechselnde Direktionalität zwischen unterschiedlich lokalisierten Dipolen nicht mehr bestimmbar. Die durch die SQUIDs eines Magnetoenzephalographen oder die durch die Elektroden eines Elektroenzephalographen gemessenen Signale können daher nicht mehr eindeutig den bestimmten Dipolen an den definierten Messorten im Phantom zugeordnet werden.

Ebenfalls im Rahmen der Erfindung wurde erkannt, dass sich auch die Algorithmen zur Analyse der Art und der Richtung der Kopplung in der Regel als sehr empfindlich gegenüber messtechnischem Rauschen erweisen, wie dies durch den oben geschilderten Vorgang selbst verursacht wird. Die gleichzeitige Erzeugung verschiedener Dipole mit nichtlinear gekoppelten Signalen bekannter Direktionalität und der anschließende Vergleich dieser Signale mit den über die Rückrechnungsalgorithmen rekonstruierten Signalverläufen ist aus diesen Gründen bisher nur unzufrieden stellend gelungen, auf der anderen Seite aber von zentraler Bedeutung für die Bewertung der Messungen.
Komplexen Fragestellungen kann auf diese Weise nicht nachgegangen werden.

Vorteilhaft können mit dem erfindungsgemäßen Phantom auch komplexe Ansteuerungsmuster nun störungsfrei erzeugt werden, so dass den genannten komplexen Fragestellungen bezüglich der Art der Kopplung und der Direktionalität nunmehr nachgegangen werden kann. Die Untersuchung der nichtlinearen Transferfunktionen des Messsystems ist mittels des erfindungsgemäßen Phantoms daher möglich.

Besonders vorteilhaft werden durch die erfindungsgemäße nichtelektrische Ansteuerung einer Vielzahl an ersten Mitteln ausschließlich die erwünschten Dipolfelder an definierten, wohl bekannten physikalischen Messorten erzeugt, ohne dass Störungen über Zuleitungen eine Lokalisation oder eine Bestimmung der Stärke oder der Wechselwirkung der Dipole zueinander erschweren.

Elektromagnetische Störungen, welche in Phantomen gemäß Stand der Technik durch die Energiezufuhr über die Zuleitungen bedingt sind, sind im erfindungsgemäßen Phantom vom Prinzip her ausgeschlossen.

In einer vorteilhaften Ausgestaltung der Erfindung umfasst das Phantom ein zweites Mittel mit mindestens einer Lichtquelle. Die Ansteuerung eines ersten Mittels erfolgt dann mit Licht.

Ein zweites Mittel umfasst hierfür vorteilhaft mindestens eine Leuchtdiode als Lichtquelle. Es ist denkbar, an Stelle einer Leuchtdiode einen Laser als Komponente eines zweiten Mittels vorzusehen. Eine Leuchtdiode ist besonders vorteilhaft, da diese preiswert verfügbar ist und auf einfache Weise durch eine Spannungsquelle angesteuert werden kann.

In einer weiteren Ausgestaltung der Erfindung umfasst ein zweites Mittel zudem mindestens einen Lichtwellenleiter als nicht elektrische Zuleitung zur Ansteuerung mindestens eines ersten Mittels. In den oder die Lichtwellenleiter wird das Licht einer oder mehrerer Lichtquellen eingekoppelt.

Das Licht tritt in einen der Lichtquelle zugeordneten Lichtwellenleiter als nicht elektrische Zuleitung zur Ansteuerung des ersten Mittels und zur lokalen Erzeugung des Dipols ein. Eine Lichtquelle und ein dieser Lichtquelle zugeordneter Lichtwellenleiter bilden vorteilhaft gemeinsam ein zweites Mittel zur Ansteuerung eines ersten Mittels aus.

Es ist aber auch denkbar, dass das Licht einer Lichtquelle in eine Vielzahl an Lichtwellenleitem zur Ansteuerung einer Vielzahl erster Mittel gleichzeitig eingekoppelt wird.

Vorteilhaft sind insbesondere eine Leuchtdiode und ein dieser Leuchtdiode zugeordneter Lichtwellenleiter preiswert erhältlich und auf einfache Weise räumlich wie auch funktionell aufeinander abstimmbar.

Auf dem offenen Ende des Lichtwellenleiters ist einer Ausgestaltung der Erfindung zur Folge besonders vorteilhaft eine konvexe Linse zur Streuung austretenden Lichts vorzugsweise aufgeklebt.
Durch die Linse wird das in gebündelter Form aus dem Lichtwellenleiter austretende Licht vorteilhaft wieder gestreut, so dass dieses ein erstes Mittel zur Erzeugung des Dipols bestrahlt und eine hohe Stromausbeute durch das erste Mittel ermöglicht.

Voraussetzung für die Verwendbarkeit und Abstimmbarkeit der verschiedenen Komponenten eines zweiten Mittels ist, dass über die nichtelektrische Zuleitung ein ausreichend starker elektrischer Strom zur Erzeugung eines ausreichend starken Dipols (-200 nAm) erzeugt werden kann.

Das aus einem Lichtwellenleiter austretende Licht trifft in einer weiteren, bevorzugten Ausgestaltung der Erfindung auf den Licht absorbierenden Teil mindestens einer Photodiode als erstes Mittel auf.

Die Photodiode erzeugt lokal einen elektrischen Strom bzw. Dipol im Phantom. Vorzugsweise ist die Photodiode geschirmt. Dies vereinfacht die Form der generierten Felder.

Mindestens eine Photodiode bildet einen Messort im Phantom aus. Die Photodiode als ein erstes Mittel umfasst vorteilhaft entweder einen Draht zur Erzeugung eines elektrischen Dipols oder eine Spule zur Erzeugung eines magnetischen Dipols. Der Widerstand des Drahtes bzw. der Spule ist bekannt.
Diese Maßnahmen bewirken Flexibilität bei der Ausgestaltung des ersten Mittels. Dadurch können eine Vielzahl qualitativ verschiedener Dipole erzeugt werden. Dies ermöglicht wiederum die Bearbeitung auch komplexer Fragestellungen der Eingangs genannten Art.

Erfindungsgemäß können daher vorteilhaft je nach Art des Phantoms und je nach Verwendungszweck sowohl Stromdipole als auch magnetische Dipole erzeugt werden.

In einer weiteren besonders bevorzugten Ausgestaltung der Erfindung bilden genau zwei oder genau drei Photodioden je einen Messort im Phantom aus.
Mehrere Photodioden je Messort sind insbesondere rechtwinklig und möglichst dicht, beispielsweise in einem Abstand von 2 bis 5 Millimetern, zueinander angeordnet. Durch diese Anordnung lässt sich auf Grund der Überlagerung der erzeugten Dipolfelder ein elektrischer bzw. magnetischer Dipol mit beliebiger Ausrichtung innerhalb einer Ebene, bei zwei Photodioden je Messort oder bei drei Photodioden je Messort sogar innerhalb des Raumes, simulieren.

Insbesondere kann bei entsprechender Ansteuerung der Photodioden ein Dipol mit zeitlich und / oder räumlich veränderbarer Orientierung simuliert werden. Jede Photodiode je Messort wird dabei vorzugsweise mittels eines eigenen Lichtwellenleiters angesteuert.

Bei zwei Photodioden je Messort kann ein Dipol mit zeitlich veränderbarer Orientierung innerhalb einer Ebene simuliert werden. Bei genau drei Photodioden je Messort im Phantom ist es möglich, einen Dipol mit auch zeitlich und / oder räumlich veränderbarer Orientierung zu simulieren.

Diese Erweiterungen optimieren die richtungsspezifische Kalibrierung des Messsystems. Bevorzugt ist hierfür die Ausgestaltung mit drei Photodioden je Messort im Phantom, da dies die größtmögliche Flexibilität bereitstellt. Aber auch bereits die Ausgestaltung mit zwei Photodioden erlaubt im Gegensatz zum Stand der Technik die Güteabschätzung bezüglich des Nachweises von z. B. rotierenden Dipolen.

Die mindestens eine Photodiode kann in einer weiteren, besonders bevorzugten Ausgestaltung der Erfindung Bereiche für die Absorption von Licht verschiedener Wellenlängen aufweisen. Derartige Zwei-Farben-Photodioden verfügen über zwei Stromausgänge, welche jeweils über Licht unterschiedlicher Farbe Wellenlänge angeregt werden.
Dadurch wird vorteilhaft bewirkt, dass auch mit nur einer Photodiode als ein erstes Mittel ein Dipol mit zeitlich wechselndem Vorzeichen (Stromrichtung) erzeugt werden kann. Dieser wird durch die zeitlich abgestimmte Einkopplung von Licht verschiedener Wellenlänge in den dieser Photodiode zugeordneten Lichtwellenleiter erzeugt.
Vorteilhaft können auf diese Weise über einen Lichtwellenleiter zwei voneinander unabhängige elektrische Ströme erzeugt werden, da der Lichtwellenleiter beide Bereiche der Photodiode zu bestrahlen vermag.

In einer weiteren Ausgestaltung sind diese Ströme antiparallel zueinander ausgerichtet und sehr dicht (z. B. ∼ 1 mm) benachbart. Auf diese Weise kann über einen Lichtwellenleiter ein Dipol mit zeitlich wechselndem Vorzeichen angesteuert werden.

Das erfindungsgemäße Phantom umfasst in einer weiteren Ausgestaltung der Erfindung eine entsprechend der Anzahl an ersten Mitteln zur Erzeugung der Dipole identische Anzahl an zweiten Mitteln zur Ansteuerung der ersten Mittel. Jedem ersten Mittel, z. B. einer Photodiode, ist dann ein zweites Mittel zugeordnet. Insbesondere sind damit aufeinander abgestimmte Lichtwellenleiter und Leuchtdioden zur Ausbildung zweiter Mittel gemeint, welche so auf die diesen zugeordneten Photodioden als erste Mittel ausgerichtet sind, dass sie mittels Bestrahlung der Photodioden die Dipole erzeugen. Das Phantom als solches ist aber keineswegs auf nur diese Ausführungsform eingeschränkt.

Im Phantom sind vorzugsweise eine Vielzahl an derartig aufeinander abgestimmten ersten und zweiten Mitteln realisiert. Das Phantom ist vorteilhaft kopfförmig ausgebildet.

Es kann eine Spannungsquelle vorgesehen sein, welche die Lichtquellen mit einer geeigneten Spannung versorgt. Die Spannungsquelle kann Bestandteil eines Dipoltreibers sein.

Sollen mehrere elektrische und / oder magnetische Dipole gleichzeitig angesteuert werden, kann das Phantom vorteilhaft ein Steuergerät umfassen. Dieses kann so ausgelegt sein, dass es mehrere Spannungen gleichzeitig generieren kann. Alternativ können auch entsprechend viele Steuergeräte angeordnet sein.

Das erfindungsgemäße Phantom ermöglicht störungsfrei die Erzeugung vieler verschiedener Dipole gleichzeitig an definierten (Mess-)Orten innerhalb des Phantoms. Bei Verwendung von Ansteuerungssignalen definierter Kopplung, z. B. einer nichtlinearen Kopplung und Direktionalität, kann ermittelt werden, inwiefern diese Kopplungseigenschaften durch das verwendete Messsystem, Magnetoenzephalograph oder Elektroenzephalograph, verzerrt bzw. verrauscht weitergegeben werden.

Vorteilhaft kann dadurch bestimmt werden, welche Kopplungsstärke und Kopplungsart mit dem Messsystem überhaupt nachgewiesen werden kann. Für diesen Nachweis ist die vollkommene Abwesenheit von Störfeldern, wie sie im Stand der Technik in Phantomen auftreten, unabdingbare Voraussetzung. Die entsprechenden Ergebnisse dienen der Kalibrierung des Messsystems, können aber auch zu dessen Optimierung verwendet werden. Ebenso kann der Fehler in der räumlichen Lokalisation gleichzeitiger Quellen über die entsprechenden Rückrechnungsalgorithmen quantifiziert werden.

Ein Verfahren, in dem ein erfindungsgemäßes Phantom vorteilhaft eingesetzt werden kann, dient der quantitativen und / oder qualitativen Ermittlung der nichtlinearen Transferfunktion eines Messsystems, wie z. B. eines Magnetoenzephalographen oder eines Elektroenzephalographen. Hierzu werden die nachfolgenden Schritte ausgeführt:
- die Dipole in einem Phantom werden über nicht elektrische Zuleitungen mittels Signalen erzeugt,
- die Sensoren des Messsystems erfassen die durch die Dipole erzeugten elektromagnetischen Felder,
- aus den Sensordaten wird die Stromdichte rekonstruiert,
- die nichtlineare Transferfunktion des Messsystems wird ermittelt.

Für die nicht elektrischen Zuleitungen werden bevorzugt Lichtwellenleiter gewählt. Signale, welche auf definierte Weise miteinander gekoppelt sind, insbesondere auf nicht-lineare Weise, gelangen in die Lichtwellenleiter. Die Dipole werden sodann an verschiedenen Messorten im Phantom erzeugt.

Aus den Sensordaten wird unter Verwendung des Algorithmus die vektorielle Stromdichte in den von dem Messsystem erfassten Voxeln rekonstruiert. Zur Rekonstruktion können auch verschiedene Algorithmen verwendet werden. Abschließend wird die nichtlineare Transferfunktion des Messsystems aus dem Vergleich der zurückgerechneten Daten mit den bekannten Signalen zur Anregung der Dipole ermittelt.

Ein weiteres Verfahren, indem das Phantom eingesetzt werden kann, dient der Überprüfung bzw. Bestimmung der Kopfposition während und / oder nach einer Magnetoenzephalographmessung. Hierzu werden optisch angesteuerte magnetische erste Mittel zur Erzeugung der Dipole an ausgewiesenen Positionen des Kopfes geklebt und zu bestimmten Zeiten magnetische Dipole exakt bekannter Größe erzeugt. Über die Rekonstruktion der Spulenposition auf Basis der Magnetoenzephalographmessung wird die Kopfposition bestimmt. Im Gegensatz zum Stand der Technik vermeidet die optische Ansteuerung die sonst vorhandenen elektromagnetischen Störungen durch die Energiezufuhr. Vorteilhaft werden dabei durch die nichtelektrische Ansteuerung Bewegungsartefakte vermieden.

Im Weiteren wird die Erfindung an Hand eines Ausführungsbeispiels und der beigefügten Figuren näher beschrieben.

Fig. 1 zeigt schematisch das Prinzip der Ansteuerung eines ersten Mittels zur Erzeugung eines Dipols mittels eines zweiten Mittels. Als erstes Mittel ist eine Photodiode 5 und als zweites Mittel eine Einheit aus Leuchtdiode 2 und zugehörigem Lichtwellenleiter 3 vorgesehen.

Fig. 2 bis Fig. 4 zeigen Ausgestaltungen an Photodioden für das erfindungsgemäße Phantom.

Eine lichtstarke weiße Leuchtdiode 2, z. B. eine Lumiled LUXEON LED, 240 Lumen, wird über eine regelbare analoge Spannungsquelle 1 a angesteuert und bestrahlt über einen Licht-wellenleiter 3 den Licht absorbierenden Teil einer Photodiode 5. Als Photodiode ist eine BPW 34 der Fa. Siemens vorgesehen.

Ein Steuergerät 1 zur Steuerung der Spannung für eine Vielzahl derartiger Leuchtdioden 2, z. B. ein PC, ist vorgesehen. Der PC 1 umfasst die Spannungsquelle 1a, vorliegend einen Digital-Analog-Wandler.

Abhängig von der über die Spannung der Spannungsquelle 1 a kontrollierten Lichtintensität der Leuchtdiode 2, erzeugt die Photodiode 5 den erwünschten Strom.

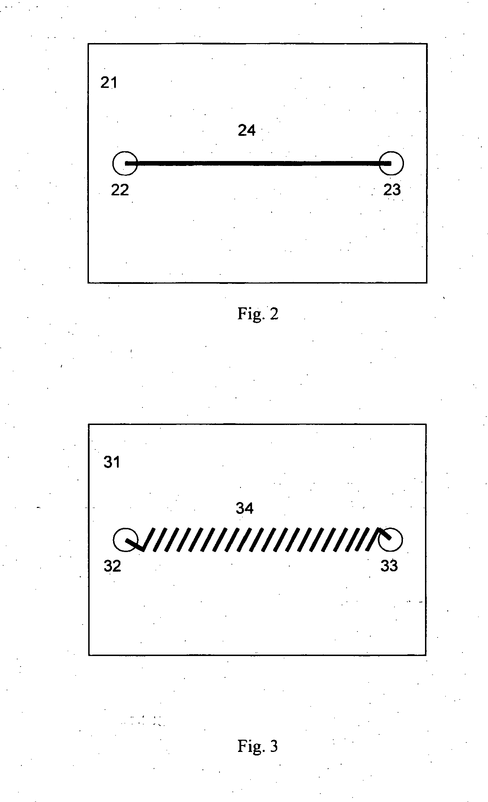

Die elektrischen Kontakte der Photodiode 5 werden zur Erzeugung eines elektrischen Dipols entweder mit einem Draht 6a gerade, oder zur Erzeugung eines magnetischen Dipols, mit einem spulenförmig gewickelten Draht 6b verbunden. Derartige Ausführungsformen der Photodioden sind in Fig. 2 bis 4 gezeigt.

In Fig. 2 sind auf der Rückseite 21 der Photodiode der elektrisch positive Kontakt 22 und der elektrisch negative Kontakt 23 der Photodiode durch den elektrisch leitenden Draht 24 miteinander verbunden.

In Fig. 3 sind auf der Rückseite 31 der Photodiode der elektrisch positive Kontakt der Photodiode 32 und der elektrisch negative Kontakt 33 der Photodiode durch den elektrisch leitenden, als Spule mit 20 Wicklungen gewickelten Draht 34 miteinander verbunden.

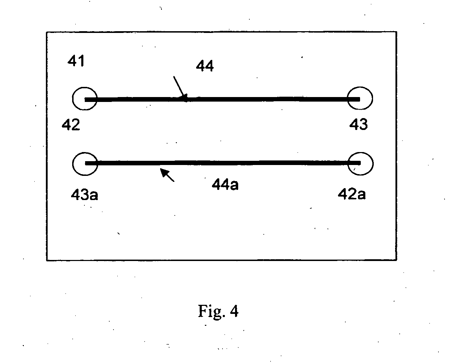

Bei einer Zweifarbendiode für das erfindungsgemäße Phantom sind die elektrischen Kontakte gemäß der Fig. 4 zur Erzeugung eines elektrischen Dipols positioniert und miteinander verbunden. Auf der Rückseite 41 der Photodiode bezeichnen die Bezugszeichen 42 und 43 den positiven und negativen Kontakt des zum ersten Wellenlängenbereich gehörenden elektrischen Ausgangs der Zweifarbenphotodiode. Bezugszeichen 42a und 43a bezeichnen den elektrisch positiven und negativen Kontakt des zum zweiten Wellenlängenbereich gehörenden elektrischen Ausgangs der Zweifarbenphotodiode. Die Kontakte sind durch die Drähte 44 und 44a in der angegebenen Weise miteinander verbunden. Alternativ kann Draht 44 und / oder 44a wiederum durch eine Spule wie in Fig. 3 gezeigt, ausgetauscht sein.

Entsprechend der Ausführungen dieser Stromleiter sowie dem Verhältnis von angelegter Spannung zum applizierten Strom der Photodiode 5 sowie dem Zeitverlauf der angelegten Spannung lassen sich bei bekanntem Widerstand die durch das Licht erzeugten magnetischen bzw. elektrischen Dipole exakt berechnen, ohne dass ein störender Einfluss durch eine Energiezuleitung berücksichtigt werden muss.

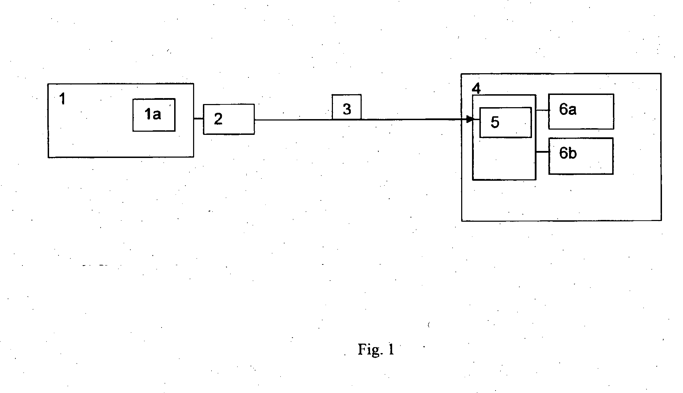

Die derartig generierten Dipole werden im Messbereich 4 des jeweiligen Messsystems, des Elektroenzephalographen oder des Magnetoenzephalographen, erfasst (s. Fig. 1).

Selbstverständlich können in dem Phantom eine Vielzahl an Leuchtdioden 2, Lichtwellenleitern 3 und speziell ausgestalteten Photodioden 5 entsprechend der Anzahl und der Art der zu erzeugenden Dipole verwendet werden und an die Spannungsquelle bzw. das Steuergerät angeschlossen werden.

## Patentansprüche

1. Verfahren zur Ermittlung der nichtlinearen Transferfunktion eines Messsystems, mit den Schritten:
- die Dipole in einem Phantom werden über nicht elektrische Zuleitungen mittels Signalen erzeugt,
- die Sensoren eines Messsystems erfassen die durch die Dipole erzeugten elektromagnetischen Felder,
- aus den Sensordaten wird die Stromdichte rekonstruiert,
- die nichtlineare Transferfunktion des Messsystems wird ermittelt.

2. Verfahren nach Anspruch 1,
**dadurch gekennzeichnet,**
**dass** als nicht elektrische Zuleitungen Lichtwellenleiter gewählt werden.

3. Verfahren nach einem der Ansprüche 1 oder 2,
bei dem ein Phantom verwendet wird, das mindestens ein erstes Mittel (5) zur Erzeugung mindestens eines Dipols umfasst,
**gekennzeichnet durch**
mindestens ein zweites Mittel (1a, 2, 3) zur Ansteuerung des mindestens einen ersten Mittels auf nicht elektrische Weise.

4. Verfahren nach Anspruch 3,
bei dem ein Phantom verwendet wird,
**dadurch gekennzeichnet,**
**dass** ein zweites Mittel mindestens eine Lichtquelle umfasst.

5. Verfahren nach Anspruch 4,
bei dem ein Phantom verwendet wird,
**dadurch gekennzeichnet,**
**dass** ein zweites Mittel mindestens eine Leuchtdiode (2) als Lichtquelle umfasst.

6. Verfahren nach einem der Ansprüche 3 bis 5,
bei dem ein Phantom verwendet wird,
**dadurch gekennzeichnet,**
**dass** ein zweites Mittel mindestens einen Lichtwellenleiter (3) umfasst.

7. Verfahren nach einem der Ansprüche 3 bis 6,
bei dem ein Phantom verwendet wird,
**dadurch gekennzeichnet,**
**dass** ein erstes Mittel mindestens eine Photodiode (5) umfasst.

8. Verfahren nach Anspruch 7,
bei dem ein Phantom verwendet wird,
**gekennzeichnet durch**
mindestens eine Photodiode (5), insbesondere genau zwei oder drei Photodioden, zur Ausbildung je eines Messorts im Phantom.

9. Verfahren nach Anspruch 7 oder 8,
bei dem ein Phantom verwendet wird, umfassend
mindestens eine Photodiode (5) mit Bereichen für die Absorption von Licht verschiedener Wellenlängen.

## Claims

1. Method of determining the non-linear transfer function of a measuring system, comprising the steps:
- the dipoles in a phantom are generated by means of signals via non-electric supply lines,
- the sensors of a measuring system detect the electromagnetic fields generated by the dipoles,
- the current density is reconstructed from the sensor data,
- the non-linear transfer function of the measuring system is determined.

2. Method according to claim 1, **characterised in that** optical waveguides are selected as the non-electric supply lines.

3. Method according to either of claims 1 or 2, in which a phantom including at least one first means (5) for generating at least one dipole is used, **characterised by** at least one second means (1a, 2, 3) for actuating the at least one first means in a non-electric manner.

4. Method according to claim 3, in which a phantom is used, **characterised in that** a second means includes at least one light source.

5. Method according to claim 4, in which a phantom is used, **characterised in that** a second means includes at least one light-emitting diode (2) as the light source.

6. Method according to one of claims 3 to 5, in which a phantom is used, **characterised in that** a second means includes at least one optical waveguide (3).

7. Method according to one of claims 3 to 6, in which a phantom is used, **characterised in that** a first means includes at least one photodiode (5).

8. Method according to claim 7, in which a phantom is used, **characterised by** at least one photodiode (5), in particular exactly two or three photodiodes each providing one measurement site in the phantom.

9. Method according to claim 7 or claim 8, in which a phantom including at least one photodiode (5) with regions for the absorption of light having different wavelengths is used.

## Revendications

1. Procédé servant à déterminer la fonction de transfert non linéaire d'un système de mesure, comprenant les étapes suivantes :
- les dipoles d'un fantôme sont produits au moyen de signaux par des conduites non électriques ;
- les capteurs d'un système de mesure détectent les champs électromagnétiques produits par les dipoles ;
- la densité de courant est reconstruite à partir des données de capteur ;
- la fonction de transfert non linéaire du système de mesure est déterminée.

2. Procédé selon la revendication 1,
**caractérisé en ce**
**que** des guides d'ondes lumineuses sont choisis en tant que conduites non électriques.

3. Procédé selon l'une quelconque des revendications 1 ou 2,
dans le cadre duquel on utilise un fantôme, qui comprend au moins un premier moyen (5) servant à produire au moins un dipole,
**caractérisé par**
au moins un deuxième moyen (1a, 2, 3) servant à commander le premier moyen au moins au nombre de un d'une manière non électrique.

4. Procédé selon la revendication 3,
dans le cadre duquel on utilise un fantôme,
**caractérisé en ce**
**qu'**un deuxième moyen comprend au moins une source de lumière.

5. Procédé selon la revendication 4,
dans le cadre duquel on utilise un fantôme,
**caractérisé en ce**
**qu'**un deuxième moyen comprend au moins comme source de lumière une diode électroluminescente (2).

6. Procédé selon l'une quelconque des revendications 3 à 5,
dans le cadre duquel on utilise un fantôme,
**caractérisé en ce**
**qu'**un deuxième moyen comprend au moins un guide d'ondes lumineuses (3).

7. Procédé selon l'une quelconque des revendications 3 à 6,
dans le cadre duquel on utilise un fantôme,
**caractérisé en ce**
**qu'**un premier moyen comprend au moins une photodiode (5).

8. Procédé selon la revendication 7,
dans le cadre duquel on utilise un fantôme,
**caractérisé par**
au moins une photodiode (5), en particulier précisément par deux ou trois photodiodes, servant à former respectivement un emplacement de mesure dans le fantôme.

9. Procédé selon la revendication 7 ou 8,
dans le cadre duquel on utilise un fantôme, comprenant
au moins une photodiode (5) dotée de zones pour l'absorption de lumière de diverses longueurs d'onde.
